# EUROPÄISCHE PATENTSCHRIFT

(11) **EP 3 642 592 B1**
(45) Veröffentlichungstag und Bekanntmachung des Hinweises auf die Patenterteilung: **21.01.2026**
(21) Anmeldenummer: 18730014.0
(22) Anmeldetag: 18.06.2018
(51) Int. Cl.: G01N 1/44, B01D 5/00, B01D 19/00, H01J 49/04, G01N 1/40, G01N 33/00

(54) **VORRICHTUNG UND VERFAHREN ZUR PARTIELLEN ÜBERFÜHRUNG EINER MEHRERE KOMPONENTEN UMFASSENDE FLÜSSIGKEITSPROBE IN DIE GASPHASE, UND VERWENDUNG DER VORRICHTUNG IN DEM VERFAHREN**
DEVICE AND METHOD FOR PARTIAL TRANSFER OF A LIQUID SAMPLE COMPRISING MULTIPLE COMPONENTS INTO THE GAS PHASE, AND USE OF THE DEVICE IN THE METHOD
DISPOSITIF ET PROCÉDÉ D'ACHEMINEMENT PARTIEL D'UN ÉCHANTILLON DE LIQUIDE COMPRENANT PLUSIEURS COMPOSANTS EN PHASE GAZEUSE, ET UTILISATION DU DISPOSITIF DANS LE PROCÉDÉ

(30) Priorität: 19.06.2017 EP 17176687
(43) Veröffentlichungstag der Anmeldung: 29.04.2020
(73) Patentinhaber: V&F Analyse- und Messtechnik GmbH, 6067 Absam (AT)
(72) Erfinder: VILLINGER, Johannes, A-6020 Innsbruck (AT); FEDERER, Werner, A-6067 Absam (AT)
(74) Vertreter: Kador & Partner Part mbB
(86) Internationale Anmeldenummer: PCT/EP2018/066128
(87) Internationale Veröffentlichungsnummer: WO 2018/234245

(56) Entgegenhaltungen:
- DE-A1- 102011 081 287
- US-A1- 2010 122 564
- US-A1- 2014 084 154
- US-B1- 9 562 430

## Beschreibung

Die Erfindung betrifft eine Vorrichtung und Verfahren zur partiellen Überführung einer mehrere Komponenten umfassende Flüssigkeitsprobe in die Gasphase. Die Erfindung betrifft ferner die Verwendung der Vorrichtung als Probenaufbereitungsvorrichtung.

Technische Prozessanlagen in der chemischen Industrie sowie in der Pharmaindustrie zur Produktion verschiedenster Produkte und Medikamente, aber auch Reinigungsanlagen für Wasser oder Lösemittel werden zurzeit im online Betrieb über einfache und integrale Prozessparameter wie Druck, Temperaturen oder Lichtabsorption etc. mittels spezifischer Sensoren überwacht. Differentielle Reaktionen in diesen Systemen, wie die Bildung von Nebenprodukten, werden jedoch erst bei der offline Produktkontrolle, die in einem Laborverfahren durchgeführt wird, erkannt. Beispielsweise ist hier die die Bildung von Nebenprodukten wie Diacetyl in der Bierfermentation oder Azotoluidin in Protein Trennsäulen, als auch die Bildung von Toxinen in anderen Prozessen zu nennen.

Als weiteres Beispiel für unerwünschte Prozesse sind Motorölverdünnungsprozesse in Verbrennungsmotoren, insbesondere Verbrennungsmotoren in Kraftfahrzeugen, zu nennen. Das Motoröl in einem Verbrennungsmotor kann durch Ethanol-hältigen Treibstoff, zum Beispiel Super E10, oder Biodiesel-hältigen Dieseltreibstoff verdünnt werden, was die Schmiereigenschaften des Motoröls signifikant ändern kann. Diese Eigenschaftsänderungen des Motoröls können sich negativ auf den Verbrennungsmotor auswirken und Schäden am Verbrennungsmotor verursachen.

Durch einen steigenden Optimierungsdruck und erhöhte Qualitätsansprüche an die Prozessanlagen werden höhere Anforderungen an eine Überwachung von oben erwähnten, unerwünschten Nebenreaktionen und mögliche Bildung von unerwünschten Verunreinigungen in den Produkten gefordert. Die aus möglichen Nebenreaktionen gebildeten Produkte oder Verunreinigungen können flüssig oder gasförmig sein.

Bei der Detektion von gasförmigen Verbindungen haben sich die Gasphasenmessgeräte, wie Flammenionisationsdetektoren, Fluoreszenzmessgeräte und Massenspektrometer, als die empfindlichsten und schnellsten Detektionssysteme etabliert.

Es bleibt dennoch das Problem bestehen, dass unerwünschte und/oder toxische Nebenprodukte unerkannt in Prozessen entstehen können, und diese nicht unverzüglich oder innerhalb kurzer Zeit erkannt werden können. Dasselbe gilt für unerwünschte Nebenprozesse, wie beispielsweise die oben erwähnte Motorölverdünnung. Im Stand der Technik werden oftmals Proben in regelmäßigen zeitlichen Abständen oder auch Stichproben genommen, welche dann üblicherweise versiegelt oder anderweitig behandelt werden bevor sie in einem Labor analysiert werden können. Durch diesen Ablauf entstehen jedoch teilweise große Zeitverluste zwischen Probennahme und Erhalt des Analysenergebnisses im Labor. Dadurch ergibt sich das Problem, dass nicht sofort auf die Bildung unerwünschter und/oder toxischer Nebenprodukte im Prozess reagiert werden kann. Solche Prozesse, wo die Probe entnommen wird und anschließend in einem Labor untersucht wird, können daher auch als "offline-Prozesse" bezeichnet werden.

Zeitaufwändig kann bei der Analyse von flüssigen Proben auch sein, wenn die unerwünschten Nebenprodukte bzw. Verunreinigungen in der Probe ebenfalls im flüssigen Zustand vorliegen. Es besteht daher auch ein Bedarf an geeigneten und verbesserten Vorrichtungen für die online Aufbereitung und online Analyse solcher Proben. US 2014/084154 A1 beschreibt eine Vorrichtung zum Bereitstellen gasförmiger Probenionen/-moleküle aus Flüssigkeitströpfchen, die Probenionen/-moleküle enthalten. DE 10 2011 081 287 A1 beschreibt ein Mikro-Probenentnahmesystem für kleine Mengen an Fluidproben zur Analyse in der Dampfphase, das mehrere integrierte Funktionseinheiten aufweist und für geringe Probenmengen bei schnellen Reaktionszeiten geeignet ist.

Es ist daher eine Aufgabe der vorliegenden Erfindung eine Vorrichtung zur partiellen Überführung einer mehrere Komponenten umfassende Flüssigkeitsprobe in die Gasphase nach Anspruch 1 bereitzustellen, das die Nachteile bekannter Vorrichtungen des Standes der Technik vermeidet.

Des Weiteren ist die Aufgabe der vorliegenden Erfindung ein Verfahren zur partiellen Überführung einer mehrere Komponenten umfassende Flüssigkeitsprobe in die Gasphase nach Anspruch 6 bereitzustellen, das die Nachteile bekannter Vorrichtungen des Standes der Technik vermeidet.

Der Erfindung liegt die Erkenntnis zugrunde, dass die oben genannten Aufgaben mit Hilfe einer Kammer gelöst werden, in der sich bei vorgegebener Temperatur rasch, d.h. innerhalb von 30 Sekunden, oder innerhalb von 20 Sekunden, oder innerhalb von 10 Sekunden, ein Gleichgewicht zwischen der Gas- und Flüssigphase einer in die Kammer eingebrachten Flüssigkeitsprobe einstellt.

Die Erfindung stellt daher eine Vorrichtung zur partiellen Überführung einer mehrere Komponenten umfassende Flüssigkeitsprobe in die Gasphase, welche
(a) eine beheizbare Kammer in welcher ein Gas-/Flüssig-Zwei- oder Mehrphasensystem erzeugt wird, die
(a1) eine Flüssigkeitszulauföffnung für den Zulauf der Flüssigkeitsprobe,
(a2) eine Flüssigkeitsablauföffnung für den Ablauf von nicht in die Gasphase überführten Flüssigkeitsbestandteilen, sowie
(a3) eine Gasphasenaustrittsöffnung zum Austritt der erzeugten Gasphase aus der Kammer aufweist, und
(b) eine Vorrichtung zur Regelung der Flussrate der Flüssigkeitsprobe in die Flüssigkeitszulauföffnung von 1 µl/min bis 3000 µl/min, umfasst,
wobei die Kammer einen oberen Bereich und einen unteren Bereich aufweist, wobei sich die Gasphasenaustrittsöffnung im oberen Bereich der Kammer befindet,
der untere Bereich mit dem oberen Bereich verbunden ist, und wobei der obere Bereich die Form eines Rotationskörpers mit konstantem Durchmesser und der untere Bereich die Form eines Rotationskörpers mit sich verkleinerndem Durchmesser hat.

Die erfindungsgemäße Vorrichtung hat den Vorteil, dass die Vorrichtung direkt an einen zu überwachenden Prozess, beispielsweise an einen Arbeits- oder Produktionsprozess, angeschlossen werden kann. Es ergibt sich hier der Vorteil, dass die erfindungsgemäße Vorrichtung "vor Ort" verwendet werden kann, insbesondere zur Aufbereitung und Vorbereitung von dem zu überwachenden Prozess entnommenen Proben. Daher kann die zu untersuchende und zu analysierende Flüssigkeitsprobe direkt aus einem solchen Prozess, beispielsweise aus einem Reaktor, einer Rohrleitung oder einem Behälter dieses Prozesses, entnommen, in die erfindungsgemäße Vorrichtung eingeleitet und dort vorbereitet bzw. aufbereitet werden.

Die erfindungsgemäße Vorrichtung ermöglicht daher in vorteilhafter Weise eine online-Probenvorbereitung und online-Probenaufbereitung von direkt einem Prozess entnommenen Flüssigkeitsproben, insbesondere für eine nachgeschaltete Analysenvorrichtung, wie beispielsweise ein Massenspektrometer.

Mit "online" im Sinne dieser Anmeldung ist gemeint, dass die gesamte Zeitdauer von Entnahme der Flüssigkeitsprobe aus einem Prozess bis Erhalt des Analyseergebnisses der Flüssigkeitsprobe aus einer Analysenvorrichtung weniger als 5 Minuten, vorzugsweise weniger als 3 Minuten und mehr bevorzugt weniger als 1 Minute beträgt. Das bedeutet, dass die direkt einem Prozess entnommene Flüssigkeitsprobe praktisch in "Echtzeit" analysiert werden kann.

Mit "online" ist aber auch gemeint, dass sowohl die Entnahme der Flüssigkeitsprobe aus dem zu überwachenden Prozess als auch die Analyse der Flüssigkeitsprobe "vor Ort" geschieht. Mit anderen Worten, die Flüssigkeitsprobe wird direkt, das heißt mittels fluider Verbindung, aus dem Prozess entnommen, in die erfindungsgemäße Vorrichtung eingeleitet und dort vor- bzw. aufbereitet, und anschließend optional in einer nachgeschalteten Analysevorrichtung, wie beispielsweise ein Massenspektrometer, analysiert.

Ein weiterer Vorteil der erfindungsgemäßen Vorrichtung besteht darin, dass die Vorrichtung lediglich geringe Volumina der Flüssigkeitsprobe aus dem zu überwachenden Prozess entnimmt, wobei die Volumina im Bereich von 10 µl bis 400 µl liegen. Die Entnahme solcher geringer Volumina beeinflusst den zu überwachenden Prozess nicht.

Ohne an Theorie gebunden sein zu wollen, erfolgt in der erfindungsgemäßen Vorrichtung entsprechend den Gesetzen der Thermodynamik eine Gleichgewichtseinstellung insofern, dass flüssige Komponenten in einer Lösung bei definierter Temperatur und Druck über der flüssigen Lösung jeweils entsprechende Partialdrücke entwickeln, die den Konzentrationen der Komponenten in den flüssigen Phasen proportional sind. Es stellt sich also ein Gas-/Flüssig-Zwei- oder Mehrphasensystem ein, welches im Gleichgewicht ist. Mit anderen Worten, die mehreren Komponenten der Flüssigkeitsprobe werden partiell in die Gasphase überführt, entsprechend ihren Konzentrationen in der flüssigen Phase der Flüssigkeitsprobe.

Ein weiterer Vorteil der erfindungsgemäßen Vorrichtung ist, dass die in die Gasphase überführten Komponenten der Flüssigkeitsprobe in der Gasphase nur in geringen Konzentrationen vorliegen, und es daher zu keinen weiteren möglichen Reaktionen der Komponenten untereinander in der Gasphase kommen kann. Daher können die gasförmigen Komponenten, falls notwendig, auch über weite Strecken zur nachgeschalteten Analysenvorrichtung, wie beispielsweise ein Massenspektrometer, transportiert werden ohne dass sich die Konzentrationen der Komponenten in der entnommenen Gasphase ändern.

Die erfindungsgemäße Vorrichtung, wie hierin in allen Ausführungsformen beschrieben, kann daher auch als Partialdruckgenerator bezeichnet werden.

Das Gas-/Flüssig-Zwei- oder Mehrphasensystem, wie oben beschrieben, stellt sich in der Kammer ein. Die Flüssigphase oder flüssige Phase dieses Zwei- oder Mehrphasensystem kann dabei einphasig, zweiphasig oder mehrphasig sein.

Die Kammer, in welcher sich das Gas/Flüssig-Zwei- oder Mehrphasensystem einstellt, befindet sich im Inneren der Vorrichtung, und kann grundsätzlich verschiedene Formen annehmen. Die Kammer weist einen oberen und einen unteren Bereich auf, wobei der untere mit dem oberen Bereich verbunden ist bzw. der obere und der untere Bereich ineinander übergehen. Der obere Bereich der Kammer hat die Form eines Rotationskörpers mit konstantem Durchmesser, und der untere Bereich der Kammer die Form eines Rotationskörpers mit sich verkleinerndem Durchmesser.

Vorzugsweise hat der Rotationskörper mit konstantem Durchmesser die Form eines Zylinders und der Rotationskörper mit sich verkleinerndem Durchmesser die Form eines Kegels oder eines Kegelstumpfs. Hierbei entspricht vorzugsweise der Durchmesser des Zylinders dem Durchmesser der Grundfläche des Kegels oder des Kegelstumpfes, d.h. die Grundfläche des Zylinders ist gleichzeitig die Grundfläche des Kegels oder des Kegelstumpfes.

In einer bevorzugten Ausführungsform der Kammer hat der obere Bereich die Form eines Zylinders und der untere Bereich die Form eines Kegels, wobei der Durchmesser des Zylinders dem Durchmesser der Grundfläche des Kegels entspricht und wobei sich die Flüssigkeitsablauföffnung an der Spitze des Kegels befindet. Damit wird erreicht, dass die Flüssigphase des Gas/Flüssig-Zwei- oder Mehrphasensystem schnell und effizient durch die Flüssigkeitsablauföffnung hindurchlaufen, und aus der Kammer bzw. der Vorrichtung entfernt werden kann.

Ist der obere Bereich der Kammer ein Rotationskörper mit konstantem Durchmesser oder hat der obere Bereich der Kammer die Form eines Zylinders, so ist der Durchmesser des Rotationskörpers beziehungsweise des Zylinders vorzugsweise zwischen 6 mm und 24 mm, mehr bevorzugt zwischen 8 mm und 22 mm, mehr bevorzugt zwischen 10 und 20 mm, und am meisten bevorzugt zwischen 12 und 18 mm.

Ist der untere Bereich der Kammer ein Rotationskörper mit sich verkleinerndem Durchmesser oder hat der untere Bereich der Kammer die Form eines Kegels oder Kegelstumpfes, so ist der Durchmesser der Grundfläche des Rotationskörpers beziehungsweise des Kegels oder Kegelstupfes vorzugsweise zwischen 6 mm und 24 mm, mehr bevorzugt zwischen 8 mm und 22 mm, mehr bevorzugt zwischen 10 und 20 mm, und am meisten bevorzugt zwischen 12 und 18 mm.

Hierbei entspricht vorzugsweise der Durchmesser des Rotationskörpers mit konstantem Durchmesser bzw. des Zylinders dem Durchmesser der Grundfläche des Rotationskörpers mit sich verkleinerndem Durchmesser bzw. des Kegels oder Kegelstumpfes.

Mit Höhe der Kammer ist der längste Abstand zwischen einem oberen Ende des oberen Bereiches bis zum untersten Ende des unteren Bereichs gemeint. Hat der obere Bereich der Kammer die Form eines Zylinders und der untere die Form eines Kegels, so ist die Höhe der Kammer der Abstand zwischen Deckfläche des Zylinders und Kegelspitze, lotrecht zur Deckfläche des Zylinders gemessen.

Die Kammer hat vorzugsweise eine Höhe zwischen 4 mm und 30 mm, mehr bevorzugt zwischen 6 mm und 28 mm, mehr bevorzugt zwischen 9 mm und 26 mm, weiter mehr bevorzugt zwischen 12 mm und 24 mm, und am meisten bevorzugt zwischen 18 mm und 22 mm.

Vorzugsweise weist die Kammer ein Volumen von 0,1 bis 25 cm³, mehr bevorzugt von 0,5 bis 20 cm³, mehr bevorzugt von 0,75 bis 15 cm³, mehr bevorzugt von 1 bis 10 cm³ und am meisten bevorzugt von 2 bis 8 cm³ auf.

Durch die Flüssigkeitszulauföffnung für den Zulauf der Flüssigkeitsprobe wird die Flüssigkeitsprobe in die Kammer eingebracht. Die Flüssigkeitszulauföffnung für den Zulauf der Flüssigkeitsprobe befindet sich vorzugsweise im unteren Bereich der Kammer, mehr bevorzugt am oder nahe dem Übergang zwischen oberem und unterem Bereich der Kammer.

Die Vorrichtung zur Regelung der Flussrate der Flüssigkeitsprobe in die Flüssigkeitszulauföffnung umfasst vorzugsweise ein Dosierventil, mehr bevorzugt ein Spindelventil. Weiter bevorzugt besteht diese Vorrichtung aus einem Ventil, mehr bevorzugt besteht sie aus einem Spindelventil. Mit dieser Vorrichtung wird die Flussrate der Flüssigkeitsprobe in die Flüssigkeitszulauföffnung der Kammer geregelt. Die Flussrate beträgt vorzugsweise 2 bis 2000 µl/min, mehr bevorzugt 5 bis 1750 µl/min, mehr bevorzugt 10 bis 1500 µl/min, mehr bevorzugt 20 bis 1000 µl/min, und am meisten bevorzugt 50 bis 500 µl/min.

Nicht in die Gasphase überführte Flüssigkeitsbestandteile der Flüssigkeitsprobe werden durch den Flüssigkeitsablauf aus der Kammer herausgeleitet. Vorzugsweise befindet sich die Flüssigkeitsablauföffnung für den Ablauf von nicht in die Gasphase überführten Flüssigkeitsbestandteilen im unteren Bereich der Kammer, mehr bevorzugt am untersten Ende des unteren Bereiches der Kammer. Die Flüssigkeitsablauföffnung befindet sich daher vorzugsweise an der Spitze des Kegels oder in der Deckfläche des Kegelstumpfes. Um die Flüssigkeit schneller und vollständig durch die Flüssigkeitsablauföffnung aus der Kammer zu saugen, kann eine Pumpe, beispielsweise eine Peristaltikpumpe, verwendet werden.

Durch die Gasphasenaustrittsöffnung werden die in die Gasphase überführten Komponenten aus der Kammer geführt. Die Gasphasenaustrittsöffnung befindet sich im oberen Bereich der Kammer, mehr bevorzugt in der oberen Hälfte des oberen Bereichs der Kammer.

Die Gasphasenaustrittsöffnung ist vorzugsweise mit einer Analysenvorrichtung verbunden. Die Analysenvorrichtung ist vorzugsweise ein Massenspektrometer, insbesondere ein zur Analyse von gasförmigen Komponenten geeignetes Massenspektrometer. Für die vorliegende Erfindung können kommerziell erhältliche bzw. im Stand der Technik bekannte Massenspektrometer, beispielsweise aus EP 0 290 711, EP 0 290 712, DE 196 28 093 und WO 02/058106, verwendet werden.

Die Verbindung zwischen Gasphasenaustrittsöffnung und Analysenvorrichtung ist vorzugsweise eine fluide Verbindung. Die fluide Verbindung ist vorzugsweise eine Kapillare, ein Rohr, ein Schlauch oder Kombinationen davon.

Vorzugsweise umfasst die Vorrichtung ferner eine Vorrichtung zur Regelung des Gasphasenstroms aus der Gasphasenaustrittsöffnung von 10 ml/min bis 500 ml/min, mehr bevorzugt 30 ml/min bis 400 ml/min, mehr bevorzugt 50 ml/min bis 300 ml/min, weiter mehr bevorzugt 70 ml/min bis 250 ml/min, und am meisten bevorzugt 100 ml/min bis 200 ml/min. Die Vorrichtung zur Regelung des Gasphasenstroms aus der Gasphasenaustrittsöffnung umfasst vorzugsweise ein Ventil bzw. besteht aus einem Ventil.

Die in der Vorrichtung befindliche Kammer ist beheizbar um in Inneren der Kammer eine einstellbare Temperatur zu erreichen. Vorzugsweise weist die Vorrichtung mindestens ein Heizelement, mehr bevorzugt mindestens zwei Heizelemente, auf, und die Beheizung der Kammer erfolgt über die die Kammer begrenzenden Wände. Vorzugsweise ist hier das Heizelement eine Heizpatrone. Mit dem Heizelement werden die die Kammer begrenzenden Wände von außen beheizt.

Vorzugsweise beträgt die Temperatur an der wärmsten Position der die Kammer begrenzenden Wände maximal 300°C, mehr bevorzugt maximal 275°C, mehr bevorzugt maximal 250°C, mehr bevorzugt maximal 225°C, weiter mehr bevorzugt maximal 200°C, weiter mehr bevorzugt maximal 175°C, und am meisten mehr bevorzugt maximal 150 °C.

Vorzugsweise beträgt die Temperatur an der wärmsten Position der der die Kammer begrenzenden Wände mindestens 20°C, mehr bevorzugt mindestens 25°C, mehr bevorzugt mindestens 30^{°}C, weiter mehr bevorzugt mindestens 35°C, weiter mehr bevorzugt mindestens 40°C, und an meisten bevorzugt mindestens 45°C.

Vorzugsweise wird die Kammer derart beheizt wird, dass sich ein Temperaturgradient vom oberen Bereich zum unteren Bereich der Kammer einstellt. Dies kann beispielweise dadurch erreicht werden, dass das Heizelement am oder nahe am oberen Bereich der Kammer positioniert wird. Somit entsteht ein Temperaturgradient in der Kammer, wobei die Temperatur im oberen Bereich höher ist als jene im unteren Bereich. Der Temperaturunterschied zwischen der wärmsten Stelle des oberen Bereichs und der kühlsten Stelle im unteren Bereich der Kammer beträgt vorzugsweise maximal 50°C, mehr bevorzugt maximal 40°C, weiter mehr bevorzugt maximal 30°C, weiter mehr bevorzugt maximal 20°C, und am meisten bevorzugt maximal 10°C.

Werden zwei, drei oder mehr Heizelemente verwendet, so ist ein Heizelement am oder nahe am oberen Bereich der Kammer positioniert, wie oben beschrieben, und die weiteren Heizelemente sind rings um die die Kammer begrenzenden Wände positioniert.

Vorzugsweise umfasst die die Kammer begrenzenden Wände einen metallischen Werkstoff. Metallische Werkstoffe sind deshalb bevorzugt, da sie gute Wärmeleitfähigkeiten besitzen, womit nicht nur die Wärmeleitung vom Heizelement zu den die Kammer begrenzenden Wänden, sondern auch die Wärmeleitung von den Wänden zur im Inneren der Kammer befindlichen Flüssigkeitsprobe rasch erfolgt. Durch geeignete Wahl des metallischen Werkstoffs kann die Einstellung des Gas-Flüssig-Gleichgewichts in der Kammer zeitlich beeinflusst werden. Je höher die Wärmeleitfähigkeit des metallischen Werkstoffs, desto schneller kann sich das Gas-Flüssig-Gleichgewicht in der Kammer einstellen. Daher umfassen die metallischen Werkstoffen Eisen, Stahl, Edelstahl, Aluminium, Kupfer, Silber sowie deren Legierungen bevorzugt, mehr bevorzugt Edelstahl. In einer bevorzugten Ausführungsform bestehen die metallischen Werkstoffe aus Eisen, Stahl, Edelstahl, Aluminium, Kupfer, Silber sowie deren Legierungen, mehr bevorzugt besteht der metallische Werkstoff aus Edelstahl.

Vorzugsweise umfasst die Kammer eine weitere Flüssigkeitszulauföffnung zur Einbringung einer verdünnenden Flüssigkeit. Die Flüssigkeitszulauföffnung zur Einbringung einer verdünnenden Flüssigkeit befindet sich vorzugsweise im unteren Bereich der Kammer, mehr bevorzugt am oder nahe dem Übergang zwischen oberem und unterem Bereich der Kammer. Vorzugsweise befindet sich die Flüssigkeitszulauföffnung zur Einbringung einer verdünnenden Flüssigkeit in derselben Ebene, also in derselben Höhe bzw. auf demselben Niveau, der Kammer wie die Flüssigkeitszulauföffnung für den Zulauf der Flüssigkeitsprobe.

Die verdünnende Flüssigkeit wird dazu verwendet, die in der Kammer befindliche Flüssigkeitsprobe zu verdünnen. Dabei ist die verdünnende Flüssigkeit vorzugsweise der "Nullstoff", also die Hauptkomponente der Flüssigkeitsprobe, wie hierin beschrieben. Durch diese zusätzliche Einbringung der Hauptkomponente in die Kammer erhöht sich die Konzentration der Hauptkomponente in der Flüssigkeitsprobe in der Kammer, und gleichzeitig wird die Konzentration der einen oder mehreren Nebenkomponenten in der Flüssigkeitsprobe in der Kammer verringert. Dies erweist sich vor allem dann als vorteilhaft, wenn die ursprüngliche Konzentration einer oder mehrerer Nebenkomponenten in der Flüssigkeitsprobe zu hoch ist, wodurch die Analyse und die quantitative Bestimmung der Nebenkomponenten fehlerbehaftet werden können. Mit ursprünglicher Konzentration im Sinne dieser Anmeldung ist die Konzentration der Haupt- und Nebenkomponenten in der Flüssigkeitsprobe vor jeglicher Verdünnung, beispielsweise durch verdünnende Flüssigkeiten und/oder (Träger-)Gase, gemeint. Mit anderen Worten entspricht die ursprüngliche Konzentration genau jener Konzentration der Flüssigkeitsprobe, wie die Flüssigkeitsprobe beispielsweise einem anderen Prozess oder einem Behälter entnommen worden ist.

Dabei umfasst die Vorrichtung vorzugsweise ferner eine Vorrichtung zur Regelung der Flussrate der verdünnenden Flüssigkeit in die Flüssigkeitszulauföffnung zur Einbringung einer verdünnenden Flüssigkeit. Diese Vorrichtung umfasst vorzugsweise ein Dosierventil, beispielsweise ein Spindelventil, mehr bevorzugt besteht diese Vorrichtung aus einem Spindelventil. Die Flussrate beträgt vorzugsweise 1 bis 3000 µl/min, mehr bevorzugt 2 bis 2000 µl/min, mehr bevorzugt 5 bis 1750 µl/min, mehr bevorzugt 10 bis 1500 µl/min, mehr bevorzugt 20 bis 1000 µl/min, und am meisten bevorzugt 50 bis 500 µl/min.

Anstelle der verdünnenden Flüssigkeit können auch andere Flüssigkeiten durch die Flüssigkeitszulauföffnung zur Einbringung einer verdünnenden Flüssigkeit eingebracht werden. Beispielsweise kann eine definierte Referenzlösung eingebracht werden um den Nullpunkt einer nachgeschalteten Analysenvorrichtung zu ermitteln. Es können aber auch eine oder mehrere Kalibrierlösungen, also Lösungen mit Komponenten bekannter Konzentrationen, eingebracht werden, um die Vorrichtung und/oder die nachgeschaltete Analysenvorrichtung zu kalibrieren.

Vorzugsweise befindet sich die Flüssigkeitszulauföffnung für den Zulauf der Flüssigkeitsprobe in die Kammer und/oder die Flüssigkeitszulauföffnung zur Einbringung einer verdünnenden Flüssigkeit in die Kammer im unteren Bereich der Kammer, mehr bevorzugt am oder nahe dem Übergang zwischen oberem und unterem Bereich der Kammer.

Vorzugsweise umfasst die Kammer ferner eine Gaszutrittsöffnung zur Einspeisung von Gasen in die Kammer. Die Gaszutrittsöffnung befindet sich dabei vorzugsweise im oberen Bereich der Kammer, mehr bevorzugt in der oberen Hälfte des oberen Bereichs der Kammer. Bevorzugt befindet sich die Gaszutrittsöffnung auf derselben Ebene wie die Gasphasenaustrittöffnung, weiter mehr bevorzugt befindet sich die Gaszutrittsöffnung auf derselben Ebene wie die Gasphasenaustrittöffnung und liegt der Gasphasenaustrittöffnung in der Kammer gegenüber.

Durch die Gaszutrittsöffnung kann Trägergas in die Kammer eingeleitet werden. Das Trägergas ist vorzugsweise ein inertes Gas wie N₂, Ar oder getrocknete Luft, besonders bevorzugt N₂. Das Trägergas dient zum Spülen der Kammer, insbesondere vor Inbetriebnahme der Kammer, um Feuchtigkeit aus der Kammer zu entfernen.

Vorzugsweise umfasst die Kammer ferner eine Vorrichtung zur Regelung der Flussrate von Gasen in die Gaszutrittsöffnung zur Einspeisung von Gasen in die Kammer. Die Flussrate beträgt vorzugsweise 50 ml/min bis 1000 ml/min, mehr bevorzugt 100 ml/min bis 900 ml/min, mehr bevorzugt 200 ml/min bis 800 ml/min, und am meisten bevorzugt 300 ml/min bis 600 ml/min.

Vorzugsweise umfasst die Kammer ferner eine Gasaustrittsöffnung (11) zum Austritt von Trägergas aus der Kammer. Durch die Gasaustrittsöffnung kann das Trägergas beim Spülen der Kammer, wie oben beschrieben, wieder aus der Kammer austreten. Die Gasaustrittsöffnung befindet sich vorzugsweise im oberen Bereich der Kammer, mehr bevorzugt auf derselben Ebene, das heißt derselben Höhe, der Kammer wie die Gaszutrittsöffnung.

Die Gasphasenaustrittsöffnung zum Austritt der erzeugten Gasphase aus der Kammer und/oder die Gaszutrittsöffnung zur Einspeisung von Gasen in die Kammer und/oder die Gasaustrittsöffnung zum Austritt von Trägergas aus der Kammer befinden sich vorzugsweise in der oberen Hälfte des oberen Bereichs der Kammer, mehr bevorzugt im oberen Drittel des oberen Bereichs der Kammer.

In einer bevorzugten Ausführungsform der Vorrichtung befinden sich die Gasphasenaustrittsöffnung zum Austritt der erzeugten Gasphase aus der Kammer und Gaszutrittsöffnung zur Einspeisung von Gasen in die Kammer und die Gasaustrittsöffnung zum Austritt von Trägergas aus der Kammer in der oberen Hälfte des oberen Bereichs der Kammer, mehr bevorzugt im oberen Drittel des oberen Bereichs der Kammer.

### Verfahren zur partiellen Überführung

Die vorliegende Erfindung betrifft ferner ein Verfahren zur partiellen Überführung einer mehrere Komponenten umfassende Flüssigkeitsprobe in die Gasphase, umfassend die Schritte
a) Einleiten der mehrere Komponenten umfassende Flüssigkeitsprobe in eine beheizbare Kammer einer Vorrichtung,
b) partielle Überführung der Flüssigkeitsprobe in die Gasphase, so dass sich in der Kammer ein Gas/Flüssig-Zwei- oder Mehrphasensystem einstellt, und
c) Entnahme der Gasphase des Gas/Flüssig-Zwei- oder Mehrphasensystem aus der Kammer durch eine Gasphasenaustrittsöffnung der Kammer

wobei die Kammer (2) einen oberen Bereich (2a) und einen unteren Bereich (2b) aufweist, wobei sich die Gasphasenaustrittsöffnung (5) im oberen Bereich (2a) der Kammer (2) befindet,
wobei der untere Bereich (2b) mit dem oberen Bereich (2a) verbunden ist, und wobei der obere Bereich (2a) die Form eines Rotationskörpers mit konstantem Durchmesser und der untere Bereich (2b) die Form eines Rotationskörpers mit sich verkleinerndem Durchmesser hat.

Alle Ausführungsformen der erfindungsgemäßen Vorrichtung wie oben beschrieben, sind auch bevorzugte Ausführungsformen der im erfindungsgemäßen Verfahrens zur partiellen Überführung einer mehrere Komponenten umfassende Flüssigkeitsprobe in die Gasphase verwendeten Vorrichtung.

Insbesondere ist die beheizbare Kammer des erfindungsgemäßen Verfahrens vorzugsweise so ausgeführt, wie oben in allen Ausführungsformen der Vorrichtung beschrieben. Mehr bevorzugt wird eine Vorrichtung umfassend die beheizbare Kammer wie oben in allen Ausführungsformen der Vorrichtung beschrieben im erfindungsgemäßen Verfahren zur partiellen Überführung einer mehrere Komponenten umfassende Flüssigkeitsprobe in die Gasphase verwendet.

Die oben beschriebenen Vorteile der erfindungsgemäßen Vorrichtung gelten analog für das erfindungsgemäße Verfahren.

Das Einleiten der mehrere Komponenten umfassende Flüssigkeitsprobe in eine beheizbare Kammer der Vorrichtung in Schritt a) erfolgt vorzugsweise über die Flüssigkeitszulauföffnung für den Zulauf der Flüssigkeitsprobe, wie oben beschrieben.

Die Temperatur der Flüssigkeitsprobe vor dem Einleiten in Schritt a) beträgt vorzugsweise 20° C bis 120°C, mehr bevorzugt zwischen 25°C und 90°C, und weiter mehr bevorzugt zwischen 30°C und 70°C.

Vorzugsweise erfolgt das Einleiten der Flüssigkeitsprobe in die Kammer in Schritt a) mit einer Flussrate von 1 µl/min bis 3000 µl/min, mehr bevorzugt von 2 µl/min bis 2000 µl/min, mehr bevorzugt 5 µl/min bis 1750 µl/min, mehr bevorzugt 10 µl/min bis 1500 µl/min, mehr bevorzugt 20 µl/min bis 1000 µl/min, und am meisten bevorzugt 50 µl/min bis 500 µl/min. Das Einleiten der Flüssigkeitsprobe in die Kammer erfolgt vorzugsweise über eine Vorrichtung, insbesondere ein Spindelventil, wie oben beschrieben. Das Einleiten der Flüssigkeitsprobe in die Kammer erfolgt dabei über eine Flüssigkeitszulauföffnung der Kammer, wie oben beschrieben.

Vorzugsweise wird das Einleiten in Schritt a) derart durchgeführt, dass maximal der gesamte untere Bereich der Kammer bzw. maximal der gesamte durch den unteren Bereich der Kammer gebildete Hohlraum mit dem flüssigen Teil oder der flüssigen Phase der Flüssigkeitsprobe gefüllt ist. Der untere Bereich der Kammer dient daher zur vollständigen Aufnahme der Flüssigkeitsprobe. Der obere Bereich der Kammer bleibt hingegen frei von flüssigen Teilen oder flüssigen Phasen der Flüssigkeitsprobe, sondern nimmt stattdessen lediglich die in die Gasphase überführten Komponenten auf. Damit wird verhindert, dass flüssige Teile oder flüssige Phase der Flüssigkeitsprobe in die Gasphasenaustrittsöffnung, und somit auch in die nachgeschaltete Analysenvorrichtung, gelangen. Das Volumen der Flüssigkeitsprobe in der Kammer beträgt üblicherweise zwischen 10 und 400 µl, vorzugsweise zwischen 20 und 350 µl, mehr bevorzugt zwischen 30 und 300 µl. Entsprechend hat der untere Bereich der Kammer, der vorzugsweise als Kegel oder Kegelstumpf wie oben beschrieben geformt ist, üblicherweise ein Volumen von 10 und 400 µl, vorzugsweise zwischen 20 und 350 µl, mehr bevorzugt zwischen 30 und 300 µl.

Vorzugsweise erfolgt das Einleiten der Flüssigkeitsprobe im Schritt a) und die Entnahme der Gasphase in Schritt c) kontinuierlich. Das bedeutet, dass ohne Unterbrechung sowohl Flüssigkeitsprobe in Schritt a) eingeleitet als auch Gasphase in Schritt c) entnommen wird.

In einer alternativen Ausführungsform erfolgt das Einleiten der Flüssigkeitsprobe im Schritt a) und die Entnahme der Gasphase in Schritt c) zeitlich getaktet. Mit zeitlich getaktet ist gemeint, dass abwechselnd ein Überführungsintervall auf ein Unterbrechungsintervall folgt. Während der Dauer des Überführungsintervalls erfolgen sowohl das Einleiten der Flüssigkeitsprobe in Schritt a) und die Entnahme der Gasphase in Schritt c). Während der Dauer des Unterbrechungsintervalls erfolgt weder ein Einleiten der Flüssigkeitsprobe in Schritt a) noch die Entnahme der Gasphase in Schritt c). Die Dauer des Überführungsintervalls kann gleich sein wie der Dauer des Unterbrechungsintervalls. Üblicherweise beträgt die Dauer des Überführungsintervall 1 Sekunde bis 60 Sekunden, vorzugsweise 2 Sekunden bis 50 Sekunden, und am meisten bevorzugt 3 Sekunden bis 40 Sekunden, und die Dauer des Unterbrechungsintervalls beträgt dabei üblicherweise zwischen 10 Sekunden und bis zu 24 Stunden, mehr bevorzugt bis zu 12 Stunden, weiter mehr bevorzugt bis zu 1h, weiter mehr bevorzugt bis zu 30 Minuten, weiter mehr bevorzugt bis zu 15 Minuten, weiter mehr bevorzugt bis zu 5 Minuten und am meisten bevorzugt bis zu 1 Minute.

Die partielle Überführung in Schritt b) erfolgt bei einer Temperatur, welche vorzugsweise in einem Bereich zwischen 20°C und 300°C, mehr bevorzugt in einem Bereich von 25°C bis 275°C, mehr bevorzugt in einem Bereich von 30°C bis 250°C, mehr bevorzugt in einem Bereich von 35°C bis 225^{°}C, mehr bevorzugt in einem Bereich von 40°C bis 200°C, und am meisten bevorzugt in einem Bereich von 45°C bis 175° C liegt. Diese im Inneren der Kammer herrschende Temperatur kann durch ein oder mehrere Heizelemente wie oben beschrieben eingestellt werden.

Vorzugsweise befindet sich das Gas/Flüssig-Zwei- oder Mehrphasensystem in der Kammer in Schritt b) zumindest zu 90% im Gleichgewichtszustand, mehr bevorzugt zumindest zu 93% im thermodynamischen Gleichgewichtszustand, weiter mehr bevorzugt zumindest zu 96% im thermodynamischen Gleichgewichtszustand, und am meisten bevorzugt zumindest zu 98% im thermodynamischen Gleichgewichtszustand. Mit Gleichgewichtszustand ist jener Zustand gemeint, der sich durch die in der Kammer gegebene Temperatur einstellt. Mit anderen Worten stellt sich bei einer vorgegebenen Temperatur für jede Komponente der Flüssigkeitsprobe nach einer gewissen Zeit ein Gleichgewicht zwischen der flüssigen und der gasförmigen Phase der Komponente in der Kammer ein.

Die Einstellung des Gleichgewichtszustandes in Schritt b) erfolgt vorzugsweise innerhalb einer Zeit von 0,5s bis 30s, mehr bevorzugt von 1s bis 20s, weiter mehr bevorzugt von 2s bis 10s, und am meisten bevorzugt zwischen 3s und 8s. Ohne durch Theorie gebunden sein zu wollen, hängt die Zeitdauer der Einstellung des Gleichgewichtszustandes in Schritt b) nicht nur von der Menge bzw. dem Volumen der in der Kammer vorliegenden Flüssigkeitsprobe ab. Bei geringer Menge bzw. geringen Volumen der Flüssigkeitsprobe, beispielsweise bei 10 bis 20 µl, erfolgt in der Regel innerhalb kurzer Zeit, wie oben angegeben, die Einstellung des Gleichgewichtszustandes in Schritt b). Die Zeitdauer hängt aber auch vom Temperaturunterschied der Flüssigkeitsprobe ab, und zwar zwischen jener Temperatur, welche die Flüssigkeitsprobe vor Einleiten in die Kammer hat, und jener gewünschten Temperatur, auf welche die Flüssigkeitsprobe in der Kammer gebracht werden soll, wie oben beschrieben. Je größer dieser Temperaturunterschied ist, desto länger dauert in der Regel die Einstellung des Gleichgewichtszustandes. Wird also die Flüssigkeitsprobe beispielsweise mit einer Temperatur von 20°C in die Kammer eingeleitet, und beträgt die gewünschte Temperatur 30°C, so erfolgt aufgrund des kleinen Temperaturunterschieds ebenfalls in der Regel innerhalb kurzer Zeit, wie oben angegeben, die Gleichgewichtseinstellung in der Kammer.

Vorzugsweise erfolgt die Entnahme der Gasphase aus der Kammer in Schritt c) mit einer Flussrate von 10 ml/min bis 500 ml/min, mehr bevorzugt von 20 ml/min bis 450 ml/min, mehr bevorzugt von 40 ml/min bis 400 ml/min, mehr bevorzugt von 60 ml/min bis 300 ml/min, und am meisten bevorzugt von 100 ml/min bis 200 ml/min. Die Entnahme der Gasphase aus der Kammer erfolgt dabei über eine Gasphasenaustrittsöffnung der Kammer, wie oben beschrieben.

Vorzugsweise wird die Flüssigphase des Gas/Flüssig-Zwei- oder Mehrphasensystem aus der Kammer durch eine Flüssigkeitsablauföffnung, wie oben beschrieben entnommen. Die Entnahme kann beispielsweise durch eine Pumpe erfolgen, wie oben beschrieben.

### Online-Verfahren

Eine Ausführungsform betrifft ein Verfahren zur online Bestimmung und Analyse von Komponenten einer mehrere Komponenten umfassende Flüssigkeitsprobe, wobei ein Verfahren zur partiellen Überführung einer mehrere Komponenten umfassende Flüssigkeitsprobe in die Gasphase, wie in allen Ausführungsformen wie oben beschrieben, verwendet wird, wobei die mehrere Komponenten umfassende Flüssigkeitsprobe einem Prozess, welcher Schritt a) vorgeschaltet ist, oder einem Behälter, welcher Schritt a) vorgeschaltet ist, entnommen wird und wobei die in Schritt c) entnommene Gasphase in eine nachgeschaltete Analysenvorrichtung eingeleitet wird.

Alle Ausführungsformen der erfindungsgemäßen Vorrichtung wie oben in allen Ausführungsformen beschrieben, sind auch bevorzugte Ausführungsformen der im erfindungsgemäßen Verfahren zur online Bestimmung und Analyse von Komponenten einer mehrere Komponenten umfassende Flüssigkeitsprobe verwendeten Vorrichtung.

Insbesondere ist die beheizbare Kammer des erfindungsgemäßen Verfahrens vorzugsweise so ausgeführt, wie oben in allen Ausführungsformen der Vorrichtung beschrieben. Mehr bevorzugt wird eine Vorrichtung umfassend die beheizbare Kammer wie oben in allen Ausführungsformen der Vorrichtung beschrieben im erfindungsgemäßen Verfahren zur online Bestimmung und Analyse von Komponenten einer mehrere Komponenten umfassende Flüssigkeitsprobe verwendet.

Alle Ausführungsformen des Verfahrens zur partiellen Überführung einer mehrere Komponenten umfassende Flüssigkeitsprobe in die Gasphase wie oben beschrieben sind auch bevorzugte Ausführungsformen des Verfahrens zur online Bestimmung und Analyse von Komponenten einer mehrere Komponenten umfassende Flüssigkeitsprobe.

Die oben beschriebenen Vorteile der erfindungsgemäßen Vorrichtung und des erfindungsgemäßen Verfahrens zur partiellen Überführung einer mehrere Komponenten umfassende Flüssigkeitsprobe in die Gasphase gelten analog für das erfindungsgemäße Verfahren zur online Bestimmung und Analyse von Komponenten einer mehrere Komponenten umfassende Flüssigkeitsprobe.

Die mehrere Komponenten umfassende Flüssigkeitsprobe wird einem Prozess, welcher Schritt a) vorgeschaltet ist, oder einem Behälter, welcher Schritt a) vorgeschaltet ist, entnommen. Die mehrere Komponenten umfassende Flüssigkeitsprobe kann dabei beispielsweise einem Reaktor, eine Rohrleitung oder einem Behälter des Prozesses entnommen werden.

Vorzugsweise ist der Schritt a) vorgeschaltete Prozess oder der Behälter mit der Vorrichtung mittels einer Fluidverbindung verbunden. Dabei umfasst die Fluidverbindung vorzugsweise ein Rohr, ein Schlauch, eine Kapillare oder Kombinationen davon.

Vorzugsweise umfasst der Schritt a) vorgeschaltete Prozess einen Verdünnungsprozess, mehr bevorzugt einen Ölverdünnungsprozess, insbesondere einen Motorölverdünnungsprozess, einen Lösemittelrückgewinnungsprozess, einen pharmazeutischen Prozess oder einen Abwasserprozess. Mehr bevorzugt ist der Schritt a) vorgeschaltete Prozess ein Verdünnungsprozess, mehr bevorzugt ein Ölverdünnungsprozess, insbesondere ein Motorölverdünnungsprozess, ein Lösemittelrückgewinnungsprozess, ein pharmazeutischer Prozess oder ein Abwasserprozess.

Ein pharmazeutischer Prozess ist beispielsweise ein Prozess zur Herstellung von Medikamenten, insbesondere flüssiger Medikamente.

Vorzugsweise beträgt das Verhältnis des Volumens der flüssigen Phase der Flüssigkeitsprobe zur gasförmigen Phase der Flüssigkeitsprobe nach Einstellen des Gleichgewichts in Schritt b), und nach optionalem Verdünnen mit verdünnender Flüssigkeit und/oder (Träger-)Gasen wie oben beschrieben, in der Kammer 1: 150, mehr bevorzugt 1 : 100.

Die entnommene Gasphase in Schritt c) wird in eine nachgeschaltete Analysenvorrichtung eingeleitet. Die Vorrichtung ist vorzugsweise mittels einer Fluidverbindung mit der nachgeschalteten Analysevorrichtung verbunden. Dabei umfasst die Fluidverbindung vorzugsweise ein Rohr, einen Schlauch, eine Kapillare oder Kombinationen davon. Die Messzeit in der nachgeschalteten Analysenvorrichtung zur Analyse und Bestimmung der in der entnommenen Gasphase enthaltenen Komponenten beträgt üblicherweise 0,05 bis 30 Sekunden, vorzugsweise 0,1 bis 15 Sekunden.

Vorzugsweise ist die nachgeschaltete Analysenvorrichtung ein Massenspektrometer, wie oben beschrieben. Im Massenspektrometer können alle Komponenten, welche sich in der im Schritt c) entnommen Gasphase befinden, qualitativ und quantitativ bestimmt werden.

Mit durchschnittlicher Verweilzeit im Sinne dieser Anmeldung ist jene Zeit gemeint, welche die Nebenkomponente(n) der Flüssigkeitsprobe durchschnittlich bzw. im zeitlichen Mittel von einer bestimmten ersten Stelle oder erstem Schritt, beispielsweise von der Entnahme der Flüssigkeitsprobe aus dem Schritt a) vorgeschalteten Prozess, zu einer bestimmten zweiten Stelle oder zweitem Schritt, beispielsweise dem Einleiten in die Schritt c) nachgeschaltete Analysenvorrichtung, benötigen.

Vorzugsweise beträgt die durchschnittliche Verweilzeit der in der Flüssigkeitsprobe enthaltenen Komponenten zwischen der Entnahme der Flüssigkeitsprobe aus dem Schritt a) vorgeschalteten Prozess und dem Einleiten in die Schritt c) nachgeschaltete Analysenvorrichtung maximal 5 Minuten, mehr bevorzugt maximal 3 Minuten und am meisten bevorzugt maximal 1 Minute.

Vorzugsweise beträgt das Molekulargewicht jeder der in die Gasphase überführten Komponenten maximal 500 Dalton, mehr bevorzugt maximal 450 Dalton, weiter mehr bevorzugt maximal 400 Dalton. Komponenten mit einem Molekulargewicht von mehr als 500 Dalton werden üblicherweise bei dem im erfindungsgemäßen Verfahren angewandten Temperaturen nicht oder in nicht messbaren Konzentrationen in die Gasphase überführt. Die Konzentration für jede in die Gasphase überführten Komponente beträgt in der Gasphase vorzugsweise zwischen 1 ppb und 1000 ppb, mehr bevorzugt zwischen 5 ppb und 750 ppb, und am meisten bevorzugt zwischen 10 ppb und 500 ppb.

In dieser Anmeldung sind mit ppb "parts per billion" gemeint, das heißt 10⁻⁹.

Vorzugsweise unterschreitet die Temperatur der entnommenen Gasphase nach Schritt c) den Taupunkt der in der Gasphase vorhandenen Komponenten nicht. Somit kondensiert keine der in der Gasphase vorhandenen Komponenten in ihren flüssigen Aggregatzustand, sondern die Komponenten erreichen in ihrem gasförmigen Zustand die nachgeschaltete Analyseneinrichtung. Das Unterschreiten der Temperatur kann beispielsweise durch thermische Isolation und/oder Beheizung der fluiden Verbindung zwischen Vorrichtung und nachgeschalteter Analyseneinrichtung verhindert werden.

Vorzugsweise beträgt die durchschnittliche Verweilzeit zwischen dem Einleiten der Flüssigkeitsprobe in die Kammer in Schritt a) und der Entnahme der Gasphase des Gas-/Flüssig-Zwei- oder Mehrphasensystem aus der Kammer in Schritt c) maximal 1 Minute, mehr bevorzugt maximal 45 Sekunden, und am meisten bevorzugt maximal 30 Sekunden. Die durchschnittliche Verweilzeit ist wie oben definiert.

Die mehrere Komponenten umfassende Flüssigkeitsprobe umfasst vorzugsweise eine Hauptkomponente und eine oder mehreren Nebenkomponenten. Die Hauptkomponente liegt dabei vor Einleiten der Flüssigkeitsprobe in die Kammer der Vorrichtung im flüssigen Zustand vor. Dabei umfasst die Hauptkomponente vorzugsweise Schmieröl, Motoröl, Abwasser, Lösungsmittel und Mischungen davon.

Die eine oder mehrere Nebenkomponenten liegen vor Einleiten der Flüssigkeitsprobe in die Kammer der Vorrichtung vorzugsweise im flüssigen Zustand in der Flüssigkeitsprobe vor, oder im gasförmigen Zustand vor und dabei in der flüssigen Hauptkomponente gelöst.

Die Nebenkomponente umfasst vorzugsweise Essigsäure, Aceton, Acetonitril, Anisol, Benzen, 1-Butanol, 2-Butanol, Butylacetat, tert-Butylmethylether, Tetrachlormethan, Chlorbenzen, Trichlormethan, Isopropylbenzen, Cyclohexan, 1,2-Dichlorethane, 1-1-Dichlorethen, 1,2-Dichlorethen, Dichlormethan, 1,2-Dimethoxyethan, N,N-Dimethylacetamid, N,N-Dimethylformamid, Dimethylsulfoxid, 1,3-Dioxan, Ethanol, 2-Ethoxyethanol, Ethylacetat, Ethylenglycol, Ethylether, Ethylformat, Methanamid, Methansäure, Heptan, Hexan, Isobutylacetat, Isopropylacetat, Methanol, 2-Methoxyethanol, Methylacetat, 3-Methyl-1-butanol, Methylbutylketon, Methylcyclohexane, Methylethylketon, Methylisobutylketon, 2-Methyl-1-propanol, N-Methylpyrrolidon, Nitromethan, Pentan, T1-Pentanol, 1-Propanol, 2-Propanol, Propylacetat, Pyridin, Sulfolan, Tetrahydrofuran, Tetralin, Toluol, 1,1,1-Trichlorethan, 1,1,2-Trichlorethen, Triethylamin, Xylen, Butan, Cyclopentan, Okten oder Mischungen davon.

Die Konzentration der Hauptkomponente in der Flüssigkeitsprobe beträgt vorzugsweise 85 Gew.% oder mehr, und die Summe aller Nebenkomponenten in der Flüssigkeitsprobe beträgt 15 Gew.% oder weniger. Mehr bevorzugt beträgt die Konzentration der Hauptkomponente in der Flüssigkeitsprobe 90% oder mehr, und die Summe aller Nebenkomponenten in der Flüssigkeitsprobe beträgt 10 Gew.% oder weniger, weiter mehr bevorzugt beträgt die Konzentration der Hauptkomponente in der Flüssigkeitsprobe 92,5 Gew. % oder mehr, und die Summe aller Nebenkomponenten in der Flüssigkeitsprobe beträgt 7,5 Gew.% oder weniger, weiter bevorzugt beträgt die Konzentration der Hauptkomponente in der Flüssigkeitsprobe 95 Gew. % oder mehr, und die Summe aller Nebenkomponenten in der Flüssigkeitsprobe beträgt 5 Gew.% oder weniger, und am meisten bevorzugt beträgt die Konzentration der Hauptkomponente in der Flüssigkeitsprobe 97,5% oder mehr, und die Summe aller Nebenkomponenten in der Flüssigkeitsprobe beträgt 2,5 Gew.% oder weniger.

Die obigen Konzentrationen beziehen sich stets auf die Konzentrationen der Haupt- und Nebenkomponente(n) in der in der Kammer vorliegenden Flüssigkeitsprobe, und zwar nach einer optionalen Einbringung einer verdünnenden Flüssigkeit und/oder nach optionalen Einspeisung von Gasen in die Kammer, wie oben beschrieben. Liegt die Konzentration der zu detektierenden Nebenkomponenten über 15 Gew.% in der Flüssigkeitsprobe in der Kammer vor so können sich, beispielsweise aufgrund von Nichtlinearitäten, Ungenauigkeiten bei der quantitativen Bestimmung dieser Nebenkomponenten in einer nachgeschalteten Analysevorrichtung ergeben. Um dem abzuhelfen kann mittels einer Vorrichtung zur Regelung der Flussrate eine verdünnende Flüssigkeit durch die weitere Flüssigkeitszulauföffnung in die Kammer eingebracht werden, wie oben beschrieben. Die verdünnende Flüssigkeit wird dazu verwendet, die in der Kammer befindliche Flüssigkeitsprobe zu verdünnen und ist vorzugsweise der "Nullstoff" bzw. die Hauptkomponente der in der Kammer befindlichen Flüssigkeitsprobe, wie oben beschrieben. Damit wird die Konzentration der zu detektierenden Nebenkomponenten in der Flüssigkeitsprobe auf 15 Gew.% oder weniger verringert, und Probleme bei der quantitativen Bestimmung dieser Nebenkomponenten vermieden.

### Verwendung

Die Erfindung betrifft ferner die Verwendung der erfindungsgemäßen Vorrichtung, wie oben in allen Ausführungsformen beschrieben, als Probenaufbereitungsvorrichtung in einem Verfahren zur partiellen Überführung einer mehrere Komponenten umfassende Flüssigkeitsprobe in die Gasphase wie oben in allen Ausführungsformen beschrieben.

Eine Ausführungsform betrifft die Verwendung der erfindungsgemäßen Vorrichtung, wie oben in allen Ausführungsformen beschrieben, als Probenaufbereitungsvorrichtung in einem Verfahren zur online Bestimmung und Analyse von Komponenten einer mehrere Komponenten umfassende Flüssigkeitsprobe wie oben in allen Ausführungsformen beschrieben.

Weitere Einzelheiten, Merkmale und Vorteile des Gegenstands der Erfindung ergeben sich aus der nachfolgenden Beschreibung der zugehörigen Figuren, in welchen beispielhaft bevorzugte Ausführungsformen der Erfindung dargestellt sind.

Darin zeigen
Figur 1 eine Aufsicht einer Ausführungsform der erfindungsgemäßen Vorrichtung zur partiellen Überführung einer mehrere Komponenten umfassende Flüssigkeitsprobe in die Gasphase,
Figur 2 einen Schnitt durch die erfindungsgemäße Vorrichtung entlang der Linie A-A in Figur 1,
Figur 3 einen Schnitt durch die erfindungsgemäße Vorrichtung entlang der Linie B-B in Figur 1.
Figur 4 einen Schnitt durch die erfindungsgemäße Vorrichtung entlang der Linie C-C in Figur 2,
Figur 5 ein Messergebnis einer online Messung von Treibstoffverdünnung gemäß einer Ausführungsform des erfinderischen Verfahrens, und
Figur 6 ein Messergebnis einer online Überwachung eines Fermentationsprozesses gemäß einer Ausführungsform des erfinderischen Verfahrens.

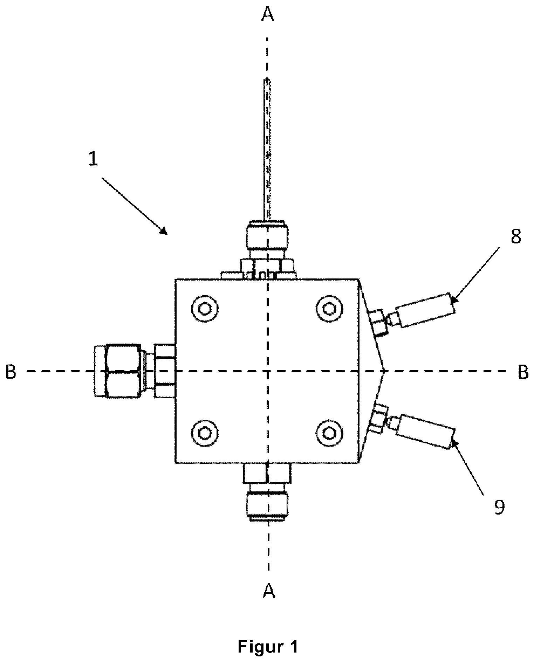

Figur 1 zeigt eine Aufsicht einer Ausführungsform der erfindungsgemäßen Vorrichtung (1) zur partiellen Überführung einer mehrere Komponenten umfassende Flüssigkeitsprobe in die Gasphase. Auf der rechten Seite der Vorrichtung (1) ist die Vorrichtung (8) zur Regelung der Flussrate der Flüssigkeitsprobe in die Flüssigkeitszulauföffnung (3) sowie die Vorrichtung (9) zur Regelung der Flussrate der verdünnenden Flüssigkeit in die Flüssigkeitszulauföffnung (6) angeordnet. Beide Vorrichtungen (8, 9) sind in diesem Ausführungsbeispiel als Spindelventile ausgeführt, mit welchem der Fluss der Flüssigkeitsprobe bzw. der verdünnenden Flüssigkeit in die Kammer (2) geregelt werden kann.

Auf der den Vorrichtungen (8,9) gegenüberliegenden Seite der Vorrichtung ist ein Auslass für Trägergas angeordnet.

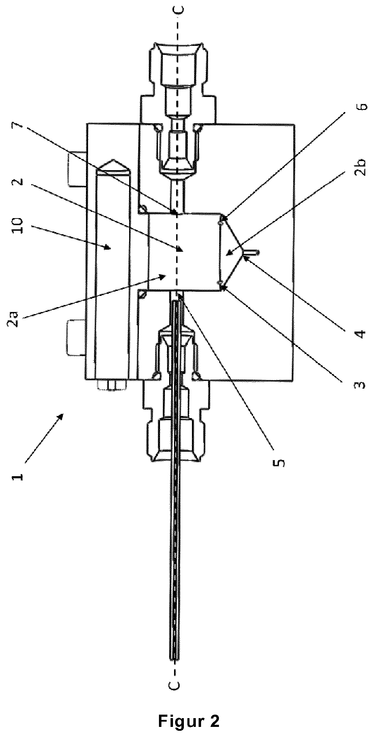

Einen Schnitt durch die erfindungsgemäße Vorrichtung entlang der Linie A-A in Figur 1 ist in Figur 2 gezeigt. Die im Inneren der Vorrichtung (1) angeordnete Kammer (2) weist einen oberen Bereich (2a) und einen an den oberen Bereich (2a) grenzenden unteren Bereichs (2b) auf. Der obere Bereich (2a) hat die Form eines geraden Kreiszylinders, während der untere Bereich (2b) die Form eines geraden Kegels hat, wobei sich an der Spitze des Kegels die Flüssigkeitsablauföffnung (4) befindet. Der Durchmesser des Kreiszylinders entspricht dem Durchmesser der kreisförmigen Grundfläche des Kegels.

Ein Heizelement (10) befindet sich oberhalb des oberen Bereiches (2a) der Kammer (2) und beheizt die Kammer über die die Kammer begrenzenden Wände, insbesondere jene den oberen Bereich (2a) der Kammer (2) begrenzenden Wände, derart, dass sich im Inneren der Kammer (2) die gewünschten Temperatur einstellt.

Im oberen Bereich (2a) weist die Kammer (2) eine Gaszutrittsöffnung (7) zur Einspeisung von Gasen in die Kammer (2), und die der Öffnung (7) gegenüberliegende Gasphasenaustrittsöffnung (5) auf. Die Gasphasenaustrittsöffnung (5) ist wie in Figur 2 gezeigt mit einer Kapillare verbunden, welche wiederrum mit einer Analysevorrichtung (nicht gezeigt) verbunden werden kann. Wie aus Figur 2 ferner ersichtlich liegen sowohl Öffnung (7) als auch Öffnung (5) auf derselben Höhe im oberen Bereich (2a) der Kammer (2). Die Kammer (2) weist ferner eine Flüssigkeitszulauföffnung (3) für den Zulauf der Flüssigkeitsprobe in die Kammer (2) sowie eine Flüssigkeitszulauföffnung (6) zur Einbringung einer verdünnenden Flüssigkeit in die Kammer (2). Beide Flüssigkeitszulauföffnungen (3, 6) sind am Übergang zwischen dem oberen Bereich (2a) zum unteren Bereich (2b) der Kammer (2) angeordnet. Mit diesen beiden Flüssigkeitszulauföffnungen (3, 6) wird der untere Bereich (2b) der Kammer (2) mit Flüssigkeitsprobe bzw. verdünnender Flüssigkeit befüllt.

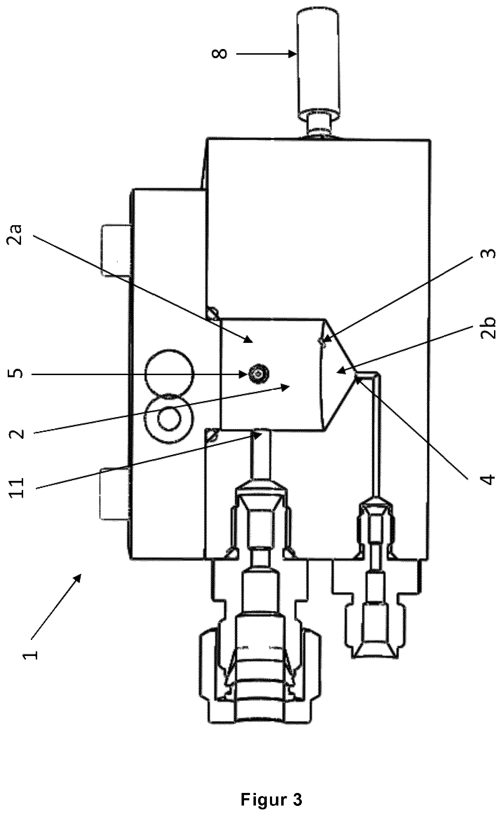

Figur 3 zeigt einen Schnitt durch die erfindungsgemäße Vorrichtung entlang der Linie B-B in Figur 1. Die Gasaustrittsöffnung (11) zum Austritt von Trägergas aus der Kammer (2) befindet sich auf selber Höhe im oberen Bereich (2a) der Kammer (2) wie die Gasphasenaustrittsöffnung (5) zum Austritt der erzeugten Gasphase aus der Kammer (2). Die Flüssigkeitsablauföffnung (4) für den Ablauf von nicht in die Gasphase überführten Flüssigkeitsbestandteilen befindet sich, wie bereits in Figur 2 gezeigt, an der Spitze des Kegels des unteren Bereichs (2b). Figur 3 zeigt auch die Flüssigkeitszulauföffnung (3) und das dieser Zulauföffnung zugeordnete Spindelventil (8).

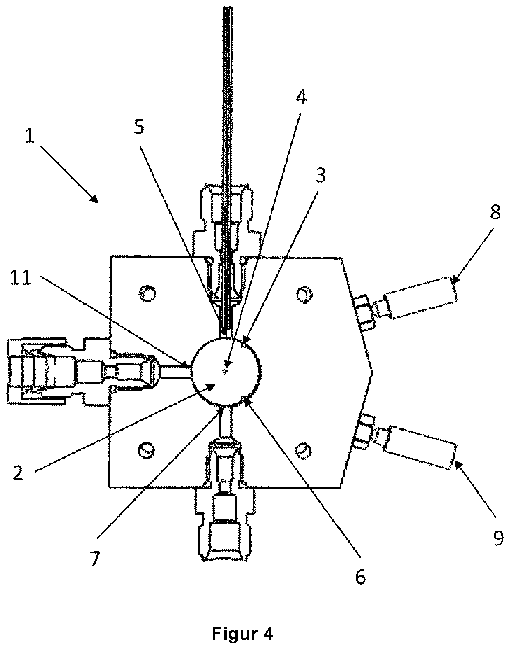

Figur 4 zeigt einen Schnitt durch die erfindungsgemäße Vorrichtung entlang der Linie C-C in Figur 2. In Figur 4 erkennt man, dass die Kammer (2) in dieser Ausführungsform zentral in der Vorrichtung (1) angeordnet ist. Wie bereits in Figuren 2 und 3 gezeigt, zeigt auch Figur 4, dass am Boden der Kammer (2) die Flüssigkeitsablauföffnung (4) mittig angeordnet ist. In Figur 4 sind ferner die Flüssigkeitszulauföffnungen (3, 6) mit ihren zugeordneten Ventilen (8, 9) zu sehen.

### Beispiel 1

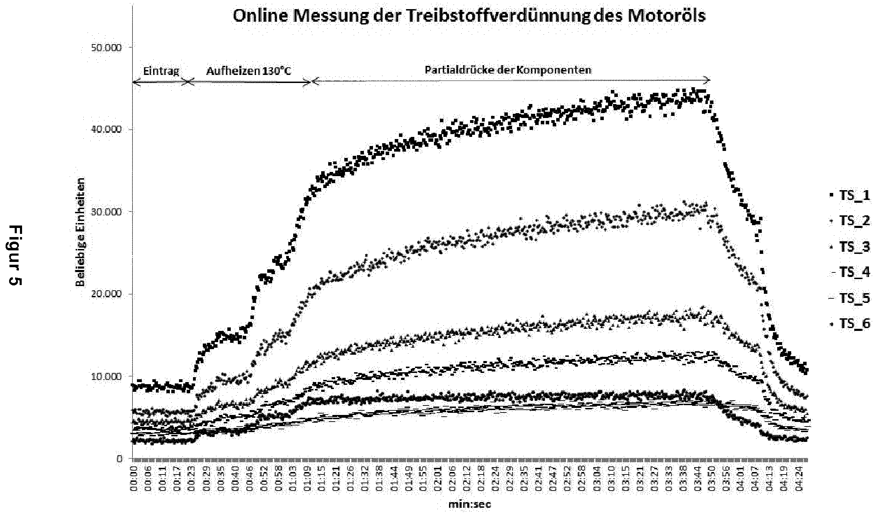

Beispiel 1 betrifft die Treibstoffverdünnung von Motoröl und soll beispielhaft das Prinzip des erfindungsgemäßen Verfahrens zur online Bestimmung und Analyse von Komponenten einer mehrere Komponenten umfassende Flüssigkeitsprobe veranschaulichen. Es wird hierbei eine Vorrichtung (1) gemäß dem oben erläuterten Ausführungsbeispiel, wie auch in Figuren 1 bis 4 gezeigt, verwendet. Ein Ionen-Molekül-Reaktions-Massenspektrometer (IMR-MS; kommerziell erhältlich von V&F Analyse- und Messtechnik GmbH) zur Bestimmung der in die Gasphase zu überführenden Komponenten der Flüssigkeitsprobe Die zu analysierende Flüssigkeitsprobe ist einer Motorölmischung von 20 ml Diesel in 5 Liter Motoröl entnommen. In Figur 5 ist der Konzentrationsverlauf von typischen Dieselkohlenwasserstoffen "TS1" bis "TS6" über die Zeit dargestellt. TS1 bis TS6 sind langkettige, d.h. C₁₂ bis C₁₆ Kohlenwasserstoffe des Dieseltreibstoffes. Bezugnehmend auf Figur 5 werden bei Sekunde 23 100 µl der Flüssigkeitsprobe mit dem Spindelventil (8) über die Flüssigkeitszulauföffnung (3) in die Kammer (2) der Vorrichtung (1) eingebracht. Das Aufheizen der in die Kammer (2) eingebrachten Flüssigkeitsprobe von 40°C auf 130°C dauert etwa 1 Minute. Anschließend werden die in die Gasphase überführten Komponenten der Flüssigkeitsprobe durch die Gasphasenaustrittöffnung (5) der Vorrichtung (1) mittels Kapillare in das Massenspektrometer zur Bestimmung und Analyse der gasförmigen Komponenten überführt. Von ca. 1 Min 21 Sek bis 3 Min 50 Sek in Figur 5 werden im Massenspektrometer die Konzentrationen der gasförmigen Komponenten gemessen. Nach Ende der Messung wird das in der Kammer (2) verbliebene Motoröl von einer Peristaltikpumpe durch die Flüssigkeitsablauföffnung (4) aus der Kammer (2) gepumpt.

### Beispiel 2

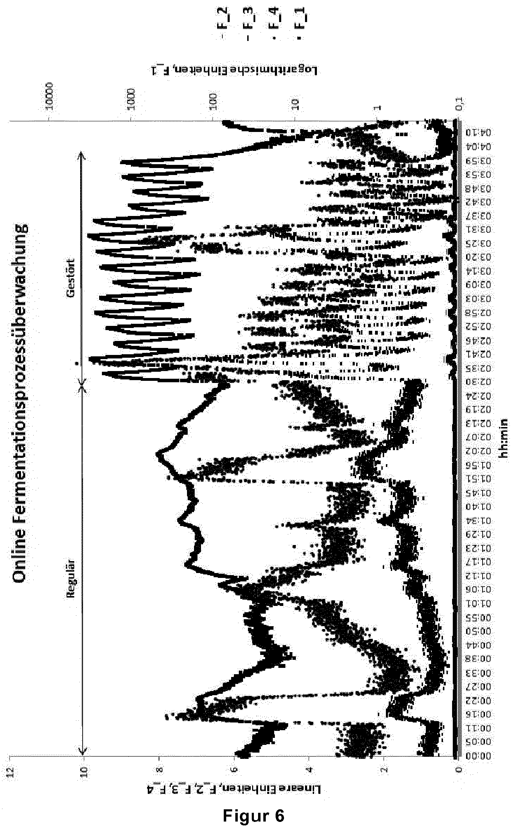

Beispiel 2 ist ein weiteres Anwendungsbeispiel des erfindungsgemäßen Verfahrens zur online Bestimmung und Analyse von Komponenten einer mehrere Komponenten umfassende Flüssigkeitsprobe veranschaulichen. Das Verfahren kann nicht nur zur Bestimmung und Analyse von Komponenten, sondern kann zugleich auch als Prozessüberwachungs-System bzw. Prozessüberwachungs-Verfahren dienen. Die online Prozessüberwachung wird anhand eines Fermentationsprozesses von Mais veranschaulicht, siehe Figur 6. Es wird in Beispiel 2 dieselbe Vorrichtung (1) inklusive Massenspektrometer wie in Beispiel 1 verwendet, wobei Flüssigkeitsproben eines Fermentationsprozesses aus dem Fermenter kontinuierlich in die Vorrichtung (1) eingebracht werden. Die gemessenen, mittels Vorrichtung (1) in die Gasphase überführten Komponenten F1 bis F4 in Figur 6 sind charakteristische Verbindungen des Fermentationsprozesses, die das richtige Funktionieren der Anlage bzw. des Fermentationsprozesses anzeigen. Bis ca. 2 h 30 sec in Figur 6 läuft der zu überwachende Fermentationsprozess normal ab. Danach werden erhebliche Schwankungen in der Konzentration dieser charakteristischen Komponenten in der Gasphase detektiert, was auf eine Störung des Prozesses hinweist.

Die beiden obigen Beispiele sollen demonstrieren, dass die erfindungsgemäße Vorrichtung und das erfindungsgemäße Verfahren zur online Bestimmung und Analyse von Komponenten geeignet sind, kontinuierliche Prozesse online, das heißt in "Echtzeit", überwachen zu können. Mit anderen Worten, Störungen in den zu überwachenden Prozessen können innerhalb kürzester Zeit, d.h. innerhalb weniger Minuten, erkannt und diagnostiziert werden.

### Bezugszeichenliste

**1** Vorrichtung zur partiellen Überführung einer mehrere Komponenten umfassende Flüssigkeitsprobe in die Gasphase,
**2** beheizbare Kammer,
**2a** oberer Bereich der Kammer (2),
**2b** unterer Bereich der Kammer (2),
**3** Flüssigkeitszulauföffnung für den Zulauf der Flüssigkeitsprobe in die Kammer (2),
**4** Flüssigkeitsablauföffnung für den Ablauf von nicht in die Gasphase überführten Flüssigkeitsbestandteilen aus der Kammer (2),
**5** Gasphasenaustrittsöffnung zum Austritt der erzeugten Gasphase aus der Kammer (2),
**6** Flüssigkeitszulauföffnung zur Einbringung einer verdünnenden Flüssigkeit in die Kammer (2),
**7** Gaszutrittsöffnung zur Einspeisung von Gasen in die Kammer (2),
**8** Vorrichtung zur Regelung der Flussrate der Flüssigkeitsprobe in die Flüssigkeitszulauföffnung (3),
**9** Vorrichtung zur Regelung der Flussrate der verdünnenden Flüssigkeit in die Flüssigkeitszulauföffnung (6),
**10** Heizelement,
**11** Gasaustrittsöffnung zum Austritt von Trägergas aus der Kammer (2)

## Patentansprüche

1. Vorrichtung (1) zur partiellen Überführung einer mehrere Komponenten umfassende Flüssigkeitsprobe in die Gasphase, welche
(a) eine beheizbare Kammer (2) in welcher ein Gas-/Flüssig- Zwei- oder Mehrphasensystem erzeugt wird, die
(a1) eine Flüssigkeitszulauföffnung (3) für den Zulauf der Flüssigkeitsprobe,
(a2) eine Flüssigkeitsablauföffnung (4) für den Ablauf von nicht in die Gasphase überführten Flüssigkeitsbestandteilen aus der Kammer (2), sowie
(a3) eine Gasphasenaustrittsöffnung (5) zum Austritt der erzeugten Gasphase aus der Kammer
aufweist,
und
(b) eine Vorrichtung (6) zur Regelung der Flussrate der Flüssigkeitsprobe in die Flüssigkeitszulauföffnung (3) von 1 µl/min bis 3000 µl/min,
umfasst, wobei die Kammer (2) einen oberen Bereich (2a) und einen unteren Bereich (2b) aufweist, wobei sich die Gasphasenaustrittsöffnung (5) im oberen Bereich (2a) der Kammer (2) befindet,
der untere Bereich (2b) mit dem oberen Bereich (2a) verbunden ist, **dadurch gekennzeichnet dass** der obere Bereich (2a) die Form eines Rotationskörpers mit konstantem Durchmesser und der untere Bereich (2b) die Form eines Rotationskörpers mit sich verkleinerndem Durchmesser hat.

2. Vorrichtung gemäß einem der vorstehenden Ansprüche, wobei die Kammer (2) ein Volumen von 0,1 bis 25 cm³ aufweist.

3. Vorrichtung gemäß einem der vorstehenden Ansprüche, wobei die Vorrichtung (1) ferner eine Vorrichtung zur Regelung des Gasphasenstroms aus der Kammer (2) von 10 ml/min bis 500 ml/min umfasst.

4. Vorrichtung gemäß einem der vorstehenden Ansprüche, wobei die Vorrichtung (1) ferner ein Heizelement (10) aufweist, und die Beheizung der Kammer (2) über die die Kammer (2) begrenzende Wände erfolgt.

5. Vorrichtung gemäß einem der vorstehenden Ansprüche, wobei die Kammer (2) eine weitere Flüssigkeitszulauföffnung (6) zur Einbringung einer verdünnenden Flüssigkeit umfasst, und/oder wobei die Kammer (2) ferner eine Gaszutrittsöffnung (7) zur Einspeisung von Trägergasen in die Kammer (2) und/oder eine Gasaustrittsöffnung (11) zum Austritt von Trägergas aus der Kammer (2) umfasst.

6. Verfahren zur partiellen Überführung einer mehrere Komponenten umfassende Flüssigkeitsprobe in die Gasphase, umfassend die Schritte
a) Einleiten der mehrere Komponenten umfassenden Flüssigkeitsprobe in eine beheizbare Kammer (2) einer Vorrichtung (1),
b) partielle Überführung der Flüssigkeitsprobe in die Gasphase, so dass sich in der Kammer (2) ein Gas/Flüssig-Zwei- oder Mehrphasensystem einstellt,
c) Entnahme der Gasphase des Gas/Flüssig-Zwei- oder Mehrphasensystem aus der Kammer (2) durch eine Gasphasenaustrittsöffnung (5) der Kammer (2),
wobei die Kammer (2) einen oberen Bereich (2a) und einen unteren Bereich (2b) aufweist, wobei sich die Gasphasenaustrittsöffnung (5) im oberen Bereich (2a) der Kammer (2) befindet,
wobei der untere Bereich (2b) mit dem oberen Bereich (2a) verbunden ist, **dadurch gekennzeichnet dass**
der obere Bereich (2a) die Form eines Rotationskörpers mit konstantem Durchmesser und der untere Bereich (2b) die Form eines Rotationskörpers mit sich verkleinerndem Durchmesser hat.

7. Verfahren gemäß Anspruch 6, wobei die partielle Überführung in Schritt b) bei einer Temperatur erfolgt, welche in einem Bereich zwischen 20°C und 300°C liegt.

8. Verfahren zur online Bestimmung und Analyse von Komponenten einer mehrere Komponenten umfassende Flüssigkeitsprobe, wobei ein Verfahren nach Ansprüchen 6 bis 7 verwendet wird, wobei die mehrere Komponenten umfassende Flüssigkeitsprobe einem Prozess, welcher Schritt a) vorgeschaltet ist, oder einem Behälter, welcher Schritt a) vorgeschaltet ist, entnommen wird und wobei die in Schritt c) entnommene Gasphase in eine nachgeschaltete Analysenvorrichtung eingeleitet wird.

9. Verfahren gemäß Anspruch 8, wobei die nachgeschaltete Analysenvorrichtung ein Massenspektrometer ist.

10. Verfahren gemäß einem der Ansprüche 8 bis 9, wobei die Kammer (2) einen oberen Bereich (2a) und einen unteren Bereich (2b) aufweist, wobei der untere Bereich (2b) mit dem oberen Bereich (2a) verbunden ist, und wobei der obere Bereich (2a) die Form eines Rotationskörpers mit konstantem Durchmesser und der untere Bereich (2b) die Form eines Rotationskörpers mit sich verkleinerndem Durchmesser hat und wobei das Einleiten in Schritt a) derart durchgeführt, dass maximal der untere Bereich (2b) der Kammer (2) mit der flüssigen Phase der Flüssigkeitsprobe gefüllt wird.

11. Verfahren gemäß einem der Ansprüche 8 bis 10, wobei die durchschnittliche Verweilzeit der in der Flüssigkeitsprobe enthaltenen Komponenten zwischen der Entnahme der Flüssigkeitsprobe aus dem Schritt a) vorgeschalteten Prozess und dem Einleiten in die Schritt c) nachgeschaltete Analysenvorrichtung maximal 5 Minuten beträgt.

12. Verfahren gemäß einem der Ansprüche 8 bis 11, wobei das Molekulargewicht jeder der in die Gasphase überführten Komponenten in Schritt b) maximal 500 Dalton beträgt.

13. Verfahren gemäß einem der Ansprüche 8 bis 12, wobei die Konzentration der in Schritt b) in die Gasphase überführten Komponenten in der Gasphase in der Kammer (2) jeweils zwischen 1 ppb und 1000 ppb beträgt.

14. Verfahren gemäß einem der Ansprüche 8 bis 13, wobei die mehrere Komponenten umfassende Flüssigkeitsprobe eine Hauptkomponente und eine oder mehreren Nebenkomponenten umfasst, wobei die Konzentration der Hauptkomponente in der Flüssigkeitsprobe 90 Gew.% oder mehr beträgt, und die Summe aller Nebenkomponenten in der Flüssigkeitsprobe 10 Gew.% oder weniger beträgt.

15. Verfahren gemäß einem der Ansprüche 6 bis 14, wobei eine Vorrichtung (1) gemäß einem der Ansprüche 1 bis 5 verwendet wird.

16. Verwendung einer Vorrichtung gemäß einem der Ansprüche 1 bis 5 als Probenaufbereitungsvorrichtung in einem Verfahren gemäß einem der Ansprüche 6 bis 15.

## Claims

1. A device (1) for partially transitioning a liquid sample comprising a plurality of components into the gas phase, said device comprising
(a) a heatable chamber (2) in which a two-phase or multi-phase gas/liquid system is generated, said chamber having
(a1) a liquid inflow opening (3) for inflow of the liquid sample,
(a2) a liquid outflow opening (4) for outflow from the chamber (2) of liquid remnants not transitioned into the gas phase, and
(a3) a gas phase discharge opening (5) for discharging the generated gas phase out of the chamber,
and
(b) a device (6) for controlling the flow rate of the liquid sample into the liquid inflow opening (3) from 1 µl/min to 3000 µl/min,
the chamber (2) having an upper region (2a) and a lower region (2b), the gas phase discharge opening (5) being present in the upper region (2a) of the chamber (2),
the lower region (2b) being connected to the upper region (2a), **characterized in that** the upper region (2a) has the shape of a rotation body having a constant diameter and the lower region (2b) has the shape of a rotation body having a decreasing diameter.

2. The device according to any one of the preceding claims, wherein the chamber (2) has a volume from 0.1 to 25 cm³.

3. The device according to any one of the preceding claims, wherein the device (1) further comprises a device for controlling the gas phase flow out of the chamber (2) from 10 ml/min to 500 ml/min.

4. The device according to any one of the preceding claims, wherein the device (1) further comprises a heating element (10) and the chamber (2) is heated by means of the walls bounding the chamber (2).

5. The device according to any one of the preceding claims, wherein the chamber (2) comprises a further liquid inflow opening (6) for introducing a diluting liquid, and/or wherein the chamber (2) further comprises a gas entry opening (7) for feeding carrier gases into the chamber (2) and/or a gas discharge opening (11) for discharging carrier gas out of the chamber (2).

6. A method for partially transitioning a liquid sample comprising a plurality of components into the gas phase, comprising the steps of
a) introducing the liquid sample comprising a plurality of components into a heatable chamber (2) of a device (1),
b) partially transitioning the liquid sample into the gas phase, so that a two-phase or multi-phase gas/liquid system arises in the chamber (2),
c) removing the gas phase of the two-phase or multi-phase gas/liquid system from the chamber (2) through a gas phase discharge opening (5) of the chamber (2),
the chamber (2) having an upper region (2a) and a lower region (2b), the gas phase discharge opening (5) being present in the upper region (2a) of the chamber (2),
the lower region (2b) being connected to the upper region (2a), **characterized in that** the upper region (2a) has the shape of a rotation body having a constant diameter and the lower region (2b) has the shape of a rotation body having a decreasing diameter.

7. The method according to claim 6, wherein the partial transitioning in step b) takes place at a temperature in a range between 20°C and 300°C.

8. A method for identifying and analyzing components of a liquid sample comprising a plurality of components online, wherein a method according to claims 6 through 7 is used, wherein the liquid sample comprising a plurality of components is taken from a process upstream of step a) or from a container upstream of step a), and wherein the gas phase removed in step c) is introduced into an analysis device connected downstream.

9. The method according to claim 8, wherein the analysis device connected downstream is a mass spectrometer.

10. The method according to any one of the claims 8 through 9, the chamber (2) having an upper region (2a) and a lower region (2b), the lower region (2b) being connected to the upper region (2a), and wherein the upper region (2a) has the shape of a rotation body having a constant diameter and the lower region (2b) has the shape of a rotation body having a decreasing diameter, and wherein the introducing in step a) is performed such that no more than the lower region (2b) of the chamber (2) is filled with the liquid phase of the liquid sample.

11. The method according to any one of the claims 8 through 10, wherein the average dwell time of the components present in the liquid sample between removing the liquid sample from the process upstream of step a) and introducing into the analysis device connected downstream of step c) is no greater than 5 minutes.

12. The method according to any one of the claims 8 through 11, wherein the molecular weight of each of the components transitioned into the gas phase in step b) is no greater than 500 daltons.

13. The method according to any one of the claims 8 through 12, wherein the concentration in the gas phase in the chamber (2) of each of the components transitioned into the gas phase in step b) is between 1 ppb and 1000 ppb.

14. The method according to any one of the claims 8 through 13, wherein the liquid sample comprising a plurality of components comprises a primary component and one or more secondary components, wherein the concentration of the primary component in the liquid sample is 90% by weight or greater, and the sum of all secondary components in the liquid sample is 10% by weight or less.

15. The method according to any one of the claims 6 through 14, wherein a device (1) according to any one of the claims 1 through 5 is used.

16. A use of a device according to any one of the claims 1 through 5 as a sample preparation device in a method according to any one of the claims 6 through 15.

## Revendications

1. Dispositif (1) pour le transfert partiel d'un échantillon liquide comprenant plusieurs composants dans la phase gazeuse, qui comprend
(a) une chambre chauffable (2) dans laquelle est produit un système à deux phases gaz/liquide ou plus, qui présente
(a1) une ouverture d'arrivée de liquide (3) pour l'arrivée de l'échantillon liquide,
(a2) une ouverture d'évacuation de liquide (4) pour l'évacuation de constituants liquides non transférés dans la phase gazeuse hors de la chambre (2), ainsi que
(a3) une ouverture de sortie de phase gazeuse (5) pour la sortie de la phase gazeuse produite hors de la chambre,
et
(b) un dispositif (6) pour réguler le débit de l'échantillon liquide dans l'ouverture d'arrivée de liquide (3) de 1 µl/min à 3 000 µl/min,
la chambre (2) présentant une zone supérieure (2a) et une zone inférieure (2b), l'ouverture de sortie de phase gazeuse (5) se trouvant dans la zone supérieure (2a) de la chambre (2),
la zone inférieure (2b) étant reliée à la zone supérieure (2a), **caractérisé en ce que** la zone supérieure (2a) a la forme d'un corps de révolution à diamètre constant et la zone inférieure (2b) a la forme d'un corps de révolution à diamètre décroissant.

2. Dispositif selon l'une quelconque des revendications précédentes, dans lequel la chambre (2) présente un volume de 0,1 à 25 cm³.

3. Dispositif selon l'une quelconque des revendications précédentes, dans lequel le dispositif (1) comprend en outre un dispositif pour réguler le débit de phase gazeuse provenant de la chambre (2) de 10 ml/min à 500 ml/min.

4. Dispositif selon l'une quelconque des revendications précédentes, dans lequel le dispositif (1) présente en outre un élément chauffant (10) et le chauffage de la chambre (2) s'effectue par l'intermédiaire des parois délimitant la chambre (2).

5. Dispositif selon l'une quelconque des revendications précédentes, dans lequel la chambre (2) comprend une autre ouverture d'arrivée de liquide (6) pour l'introduction d'un liquide de dilution, et/ou dans lequel la chambre (2) comprend en outre une ouverture d'entrée de gaz (7) pour l'injection de gaz vecteurs dans la chambre (2) et/ou une ouverture de sortie de gaz (11) pour la sortie de gaz vecteur hors de la chambre (2).

6. Procédé pour le transfert partiel d'un échantillon liquide comprenant plusieurs composants dans la phase gazeuse, comprenant les étapes suivantes :
a) l'introduction de l'échantillon liquide comprenant plusieurs composants
dans une chambre chauffante (2) d'un dispositif (1),
b) le transfert partiel de l'échantillon liquide dans la phase gazeuse, de telle sorte qu'un système à deux phases gaz/liquide ou plus s'établit dans la chambre (2),
c) le prélèvement de la phase gazeuse du système à deux phases gaz/liquide ou plus hors de la chambre (2) par une ouverture de sortie de phase gazeuse (5) de la chambre (2),
la chambre (2) présentant une zone supérieure (2a) et une zone inférieure (2b), l'ouverture de sortie de phase gazeuse (5) se trouvant dans la zone supérieure (2a) de la chambre (2),
la zone inférieure (2b) étant reliée à la zone supérieure (2a), **caractérisé en ce que** la zone supérieure (2a) a la forme d'un corps de révolution à diamètre constant et la zone inférieure (2b) a la forme d'un corps de révolution à diamètre décroissant.

7. Procédé selon la revendication 6, dans lequel le transfert partiel à l'étape b) s'effectue à une température qui se situe dans une plage comprise entre 20 °C et 300 °C.

8. Procédé pour la détermination et l'analyse en ligne de composants d'un échantillon liquide comprenant plusieurs composants, un procédé selon les revendications 6 et 7 étant utilisé, l'échantillon liquide comprenant plusieurs composants étant prélevé dans un processus en amont de l'étape a) ou dans un récipient en amont de l'étape a), et la phase gazeuse prélevée à l'étape c) étant introduite dans un dispositif d'analyse en aval.

9. Procédé selon la revendication 8, dans lequel le dispositif d'analyse en aval est un spectromètre de masse.

10. Procédé selon l'une quelconque des revendications 8 et 9, dans lequel la chambre (2) présente une zone supérieure (2a) et une zone inférieure (2b), la zone inférieure (2b) étant reliée à la zone supérieure (2a), et la zone supérieure (2a) ayant la forme d'un corps de révolution de diamètre constant et la zone inférieure (2b) ayant la forme d'un corps de révolution de diamètre décroissant, et l'introduction à l'étape a) étant effectuée de telle sorte qu'au maximum la zone inférieure (2b) de la chambre (2) soit remplie avec la phase liquide de l'échantillon liquide.

11. Procédé selon l'une quelconque des revendications 8 à 10, dans lequel le temps de séjour moyen des composants contenus dans l'échantillon liquide entre le prélèvement de l'échantillon liquide à partir du processus en amont de l'étape a) et l'introduction dans le dispositif d'analyse en aval de l'étape c) est de 5 minutes au maximum.

12. Procédé selon l'une quelconque des revendications 8 à 11, dans lequel la masse moléculaire de chacun des composants transférés dans la phase gazeuse à l'étape b) est au maximum de 500 daltons.

13. Procédé selon l'une quelconque des revendications 8 à 12, dans lequel la concentration dans la phase gazeuse dans la chambre (2) des composants transférés dans la phase gazeuse à l'étape b) est comprise entre 1 ppb et 1 000 ppb.

14. Procédé selon l'une quelconque des revendications 8 à 13, dans lequel l'échantillon liquide comprenant plusieurs composants comprend un composant principal et un ou plusieurs composants secondaires, la concentration du composant principal dans l'échantillon liquide étant de 90 % en poids ou plus, et la somme de tous les composants secondaires dans l'échantillon liquide étant de 10 % en poids ou moins.

15. Procédé selon l'une quelconque des revendications 6 à 14, dans lequel on utilise un dispositif (1) selon l'une quelconque des revendications 1 à 5.

16. Utilisation d'un dispositif selon l'une quelconque des revendications 1 à 5 comme dispositif de préparation d'échantillons dans un procédé selon l'une quelconque des revendications 6 à 15.
